# EUROPEAN PATENT SPECIFICATION

(11) **EP 2 925 291 B1**
(45) Date of publication and mention of the grant of the patent: **28.03.2018**
(21) Application number: 13803298.2
(22) Date of filing: 06.08.2013
(51) Int. Cl.: A61Q 19/00, A61K 8/97

(54) **OLIVE SEED JUICE AND A METHOD FOR PREPARATION**
OLIVENKERNSAFT UND HERSTELLUNGSVERFAHREN DAFÜR
JUS DE NOYAUX D'OLIVES ET PROCÉDÉ DE PRÉPARATION

(30) Priority: 05.09.2012 TR 201210172
(43) Date of publication of application: 07.10.2015
(73) Proprietor: Özen, Sebnem, Konak/Izmir (TR)
(72) Inventor: Özen, Sebnem, Konak/Izmir (TR)
(74) Representative: Yamankaradeniz, Kemal
(86) International application number: PCT/TR2013/000256
(87) International publication number: WO 2014/039019

(56) References cited:
- WO-A1-83/00433
- US-A- 2 475 419
- G. RODRIGUEZ: "Olive stone an attractive source of bioactive and valuable compounds", BIOSRESOURCE TECHNOLOGY, vol. 99, 2008, pages 5261-5269, XP002723372,

## Description

### The Related Art

The invention relates to cosmetics sector. The invention particularly relates to juice obtained from olive seed ash to be used in place of aqua and distilled water in the cosmetics sector and to the method of obtaining this juice.

### The Prior Art

Nowadays, a large portion of the society use cosmetic products in order to feel better. These products, besides making people feel better and elegant; their activities have also been increasing. In recent years, these products are accepted to be biologically acting compounds.

Beauty has been important in all ages and thus various materials have been used for centuries. However, as a result of the close interest of men, besides women, for cosmetics in recent years, use of cosmetics and personal care products have been significantly increasing.

In studies made about the cosmetics sector, effective formulations are obtained by means of synthesizing and clarifying various new molecules that are already found in the nature, but not known widely. Continuous increase of the knowledge about biochemistry and skin physiology, high speed biotechnological improvement, and discovery of quite effective new molecules led to great improvements in the cosmetics field.

Cosmetic is a concept covering the products, care, and treatment in order to make a person more appealing or eliminate appearance problems. Make up is a subfield of cosmetics, which comprises the cosmetic products and processes such as lipstick, mascara, foundation cream etc. to make the face and the face area of a person prettier or give a different appearance to a person. Besides make up materials, hair dye, hair spray, hair gel, perfume, bath salt, body fat and creams etc. numerous other products are also included in the category of cosmetics.

Vegetal, animal, and mineral fats, waxes, fatty acids, auxiliary and active ingredients, and water etc. various raw materials are used in obtaining cosmetic products.

Substances used in cosmetics have impact on the cellular events on the skin and they provide healthier and younger appearance by stimulating the regeneration, repair and control mechanisms.

Nowadays, water known as distilled water (aqua) and mineral water are used in obtaining all kinds of cosmetic products. This water is not different from normal drinking water. And in the product, it only has the benefits of normal water. It does not provide any benefit that would improve any characteristics of the cosmetic product.

Generally various chemical substances are used in order to provide more efficient and versatile cosmetic products. This situation increases the cost of cosmetic products, while increasing their harm on the skin. Relevant improvements in the prior art are given below.

Patent publication no TR 2002 02488 relates to highly functional water comprising titanium and the device and method for producing this water. The invention is formed of method and device for efficiently producing novel titanium molten water comprising molten titanium in molten state and has a physiologically activating function. Said highly functional water is used in health products, medical products or cosmetic products.

As a result, the above said drawbacks and the inadequacy of the prior solutions about the subject have necessitated improvement in the related technical field.

### Brief Description of the Invention

The invention is formed by being inspired from the prior art and aims to solve the above said problems.

The primary purpose of the invention is to obtain high bioavailability juice to be used in cosmetics field.

The purpose of the invention is to obtain juice that can be used in all kinds of cosmetic products. Another purpose of the invention is to obtain a juice, which can be used in place of distilled water and mineral water in cosmetic products, and which may improve the performance of the cosmetic product.

In order to achieve the above said purposes, the invention comprises olive seed juice used in the cosmetics field in place of distilled and mineral water.

In order to achieve the above said purposes, the olive seed juice of the invention is the juice obtained from olive seed ash.

In order to achieve the above said purposes, the invention is the method of obtaining olive seed juice to be used in the cosmetics field, and it comprises the operation steps of:
- converting olive seed into ash,
- boiling the ash by mixing with water,
- incubation of the boiled water, and
- separation of the water and the ash.

### Detailed Description of the Invention

The invention relates to juice obtained from olive seed ash to be used in place of aqua and distilled water in the cosmetics sector and to the method of obtaining this juice. Below, certain characteristics and benefits of olive seed are briefly given.

### Olive Seed

Olive trees are the most durable trees in the world. Their long life is mostly related to the production of a substance called "oleuropein", which gives them resistance against sickness and pests.

Oleuropein found in all parts of the olive tree and having a hot-bitter taste is removed during processing of olive. "Elenolic acid" found in oleuropein and "calcium elenolate", which is a derivative of oleuropein has the characteristic of avoiding various groups of microorganisms.

Just like grape seed, olive seed also comprises antioxidant, anticarcinogenic, and anti-inflammatory agents. Substances that render olive oil different from other oils are found in olive seed in the densest state. If both ends of an olive seed are lightly rasped, it can be seen that the inner part of the seed is hollow and it comprises quite dense oil. Concentration of these substances in the seed reaches up to 80%. Olive seeds can also be consumed after milling.

Olive seed is the easiest food to digest. Digestive juice decomposes olive seed in a very short time and obtains pure, healing olive oil and the remaining pulp repairs and eases the bowels. Researches about the subject have been carried on since 1985.

People with acid indigestion say that they feel relieved after eating olive seed. It is also known that olive seed reduces the risk of formation of carcinogenic cell in internal organs. People eating olive seed do not have hemorrhoid problem. It prevents constipation and hemorrhoid bleeding. It is also good for ulcer, gastritis, indigestion etc. stomach problems. It regulates bowels and gastrointestinal tract and heals bowel inflammation. It prevents prostate formation. It prevents hypertension. It eliminates liver inflammation. It prevents arteriosclerosis and infarction. It balances cholesterol. It is good for patients having diabetes.

Olive is used and known for centuries and has benefit in every sense. However, up until today, obtaining juice from olive seed ash and using this juice in the cosmetics field hasn't been considered. Juice with ash has been used as cleaner since ancient times. However, especially incineration of olive seed and method of obtaining water/juice from this ash hasn't been applied.

**The method of obtaining cell regenerative juice according to the invention comprises the operation steps of:**
- Converting olive seed into ash,
- boiling the ash by mixing with water, and
- Incubation following the boiling operation, and
- Separation of the water and the ash.

Olive seed is first incinerated in a closed or open medium like oven. Seeds are completely brought into ash form as a result of incineration. The ash obtained from olive seed is taken into a container and water is added. 5 liters of normal drinking water is added to about 500-1000 g of ash, although the ratio may change according to the cosmetic formulation used. Since these ratios are not fixed, it is also possible to obtain the juice of the invention with desired density.

Heating operation is made following addition of water onto ash. Heating operation is ended when boiling starts in the water with ash, and then it is left for conditioning. In this way, after conditioning, the ash part is settled at the bottom of the container and the liquid part can easily be taken to another container from the top part.

The most significant characteristic of the juice obtained from olive seed is having all the same benefits with olive seed. Among these benefits, the most important one may be having cell regenerative characteristic.

The juice of the invention is used in place of distilled water in all cosmetic and cosmeceutical fields.

With the method of the invention, juice is obtained, which can be used in all kinds of cosmetic formulations as a base in place of distilled water. The most significant characteristic of the water obtained is bringing the product performance to the top level in all kinds of cosmetic formulations. Having the top performance in cosmetic and dermatological means, the juice of the invention is considered to be superior to normal distilled and mineral water.

## Claims

1. Method of obtaining olive seed juice to be used in the field of cosmetics, **characterized in that** it comprises the operation steps of:
- converting olive seed into ash,
- boiling the ash by mixing with water, and
- separation of the water and the ash.

2. Olive seed juice obtainable from the method defined in claim 1, **characterized in that** the olive seed juice is the juice obtained from olive seed ash.

3. Use of the olive seed juice obtainable from the method defined in claim 1, in the field of cosmetics as a substitute for distilled and mineral water.

## Patentansprüche

1. Verfahren zum Erlangen von Olivensamensaft zur Verwendung auf dem Gebiet der Kosmetik, **dadurch gekennzeichnet, dass** es die folgenden Betriebsschritte umfasst:
- Verwandeln von Olivensamen in Asche,
- Sieden der Asche durch Mischen mit Wasser, und
- Trennen des Wassers und der Asche.

2. Olivensamensaft, der durch das in Anspruch 1 definierte Verfahren erlangt werden kann, **dadurch gekennzeichnet, dass** Olivensamensaft der Saft ist, der aus Olivensamenasche erlangt wird.

3. Verwenden des Olivensamensafts, der durch das in Anspruch 1 definierte Verfahren erlangt werden kann, auf dem Gebiet der Kosmetik als ein Ersatz für destilliertes Wasser und Mineralwasser.

## Revendications

1. Procédé pour obtenir du jus de graines d'olives devant être utilisé dans le domaine des cosmétiques, **caractérisé en ce qu'**il comprend les étapes consistant à :
- convertir la graine d'olive en cendre,
- faire bouillir la cendre en la mélangeant à de l'eau, et
- séparer l'eau et la cendre.

2. Jus de graines d'olives pouvant être obtenu par le procédé défini dans la revendication 1, **caractérisé en ce que** le jus de graines d'olives est le jus obtenu de la cendre de graines d'olives.

3. Utilisation du jus de graines d'olives pouvant être obtenu par le procédé défini dans la revendication 1 dans le domaine des cosmétiques pour remplacer l'eau distillée et minérale.
